# EUROPEAN PATENT SPECIFICATION

(11) **EP 1 354 869 B1**
(45) Date of publication and mention of the grant of the patent: **28.07.2004**
(21) Application number: 03008087.3
(22) Date of filing: 15.04.2003
(51) Int. Cl.: C07C 213/06, C07C 231/12

(54) **Process for producing quaternary ammonium salt having ester group**
Verfahren zur Herstellung quaternärer Ammoniumsalze, die eine Estergruppe haben
Procédé de préparation de sels d'ammonium quaternaires ayant un groupe ester

(30) Priority: 18.04.2002 JP 2002115600
(43) Date of publication of application: 22.10.2003
(73) Proprietor: KAO CORPORATION, Chuo-ku, Tokyo (JP)
(72) Inventor: Tomifuji, Takeshi, Wakayama-shi, Wakayama (JP); Sakaguchi, Akira, Wakayama-shi, Wakayama (JP); Ohtawa, Yasuki, Wakayama-shi, Wakayama (JP); Katoh, Tohru, Wakayama-shi, Wakayama (JP)
(74) Representative: HOFFMANN - EITLE

(56) References cited:
- EP-A- 1 239 024
- WO-A-91/01295
- US-A- 6 166 232
- YASUYUKI TAKAYANAGI ET AL: "Manufacture of unsaturated quaternary ammonium salts" CHEMICAL ABSTRACTS + INDEXES, AMERICAN CHEMICAL SOCIETY. COLUMBUS, US, vol. 15, no. 122, 10 April 1995 (1995-04-10), page 994 XP002041743 ISSN: 0009-2258

## Description

### Technical Field

This invention relates to a novel process for producing a quaternary ammonium salt having an ester group in the molecule thereof, which is useful as a surfactant and a softening agent for textile fabrics, hair, etc.

### Background Art

Generally, a conventional process for producing a quaternary ammonium salt having an ester group useful as a softening agent for textile fabrics, hair, etc. involves esterification of a tertiary amine having a hydroxyl group with a fatty acid or a fatty ester, to give a tertiary amine having an ester group, and then quaternarization of the obtained amine compound having an ester group with a quaternarizing agent such as an alkyl halide or dialkyl sulfate in the presence of a solvent. In this process, however, the temperature for the esterification reaction is as high as 150°C or more, and a catalyst such as acid or base is generally used, so there occur anxieties about deterioration in qualities (color, odor) due to the temperature and the catalyst, and thus additives such as an antioxidant are often necessary. In the quaternarization, the rate of reaction is decreased by electron-withdrawing feature of the ester group, and further the purity of the quaternary ammonium salt is decreased owing to formation of an amine salt resulting from decomposition of the quaternarizing agent. To solve these problems, a method of quaternarization in the coexistence of an alkali is known, but there is a disadvantage that when an ester group is present, its hydrolysis proceeds thus making the method unusable.

For producing a quaternary ammonium salt having an ester group, therefore, there is a demand for a method free from the above disadvantage.

A proposed method involves quaternarization of a tertiary amine having a hydroxyl group with a quaternarizing agent such as an alkyl halide or dialkyl sulfate to give a quaternary ammonium salt having a hydroxyl group, and then esterification of the obtained quaternary ammonium salt to produce a quaternary ammonium salt having an ester group. For example, JP-W 2000-512287 describes a method which involves esterification of a quaternary ammonium salt having a hydroxyl group with a fatty acid in large excess relative to a quaternary ammonium salt having a hydroxyl group by using phosphorus oxyacid or a derivative thereof. In this method, however, a large excess amount of the fatty acid remains contained in the product after the reaction, but should be removed in some cases by a film distiller. It is moreover necessary to add the catalyst and, after the reaction, inactivate it by neutralization etc., removing water at a reduced pressure. Such an esterification is too complicated in procedures. Accordingly, there is a need for a production process wherein neither catalyst nor reduced pressure are needed, a simple set of facility may be used and then the amount of the fatty acid remaining after the reaction can be minimized.

### Disclosure of Invention

The object of this invention is to provide a process for producing a quaternary ammonium salt having an ester group easily with less byproduct.

This invention provides a process for producing a quaternary ammonium salt in which all or part of hydroxyl groups of the below shown quaternary ammonium salt (1) are esterified, which comprises reacting a quaternary ammonium salt (quaternary ammonium salt (1)) represented by formula (1): 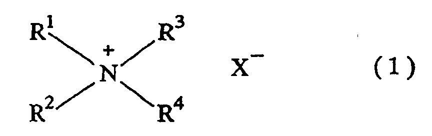 wherein X⁻ represents an anion, R¹ represents a C₁₋₆ alkyl group or a benzyl group, R² represents a C₁₋₆ alkyl group or -(CₙH₂ₙO)_{q}-H, R³ represents -(CₘH₂ₘO)ᵣ-H, R⁴ represents -(CₚH₂ₚO)ₛ-H or -CₜH₂ₜ-Y-COR⁵, n, m, p and t are the same or different and each represents an integer of 2 to 4, q, r and s each represents an integer of 1 to 5, Y represents -O- or -NH-, and R⁵ represents a C₇₋₃₅ linear or branched alkyl or alkenyl group,
with a fatty acid anhydride (fatty acid anhydride (2)) represented by formula (2) : 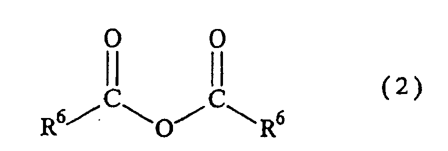 wherein R⁶ groups are the same or different and each represents a C₇₋₃₅ linear or branched alkyl or alkenyl group.

### Detailed Description of Invention

In the quaternary ammonium salt (1), R¹ is preferably a C₁₋₃ alkyl group or a benzyl group, particularly preferably a methyl group. R² is preferably a C₁₋₃ alkyl group, a hydroxyethyl group or a hydroxypropyl group, particularly preferably a methyl group or a hydroxyethyl group. R³ is preferably -(C₂H₄O)ᵣ-H (r is 1 to 5), particularly preferably a hydroxyethyl group. R⁴ is preferably -CₜH₂ₜ-Y-COR⁵ wherein t is preferably 2, and Y is -NH- or -O-. R⁵ is preferably a C₇₋₃₅ linear or branched alkyl or alkenyl group, more preferably a C₁₁₋₂₂ alkyl or alkenyl group, particularly preferably that residue of tallow fatty acid, palm fatty acid, palm kernel oil fatty acid, palm stearic acid or a hardened fatty acid thereof from which a carboxyl group was removed.

The quaternary ammonium salt (1) is preferably a compound represented by formula (3) : 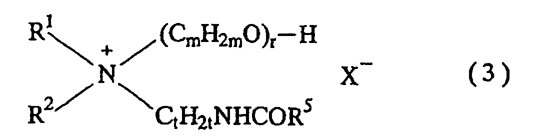 wherein X⁻, R¹, R², m, r, t and R⁵ have the same meanings as defined above.

Preferable examples of the compound represented by formula (3) include:

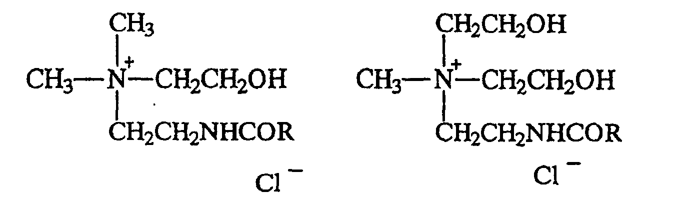

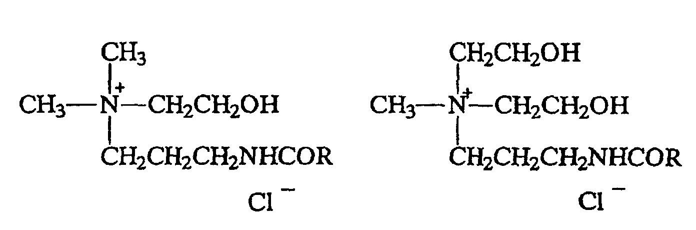

It is also preferable that the quaternary ammonium salt (1) is a compound (3') having the above shown formula (1) wherein X⁻ represents an anion, R¹ represents a C₁₋₆ alkyl group or a benzyl group, R² represents -(CₙH₂ₙO)_{q}-H, R³ represents -(CₘH₂ₘO)ᵣ-H, R⁴ represents -(CₚH₂ₚO)ₛ-H or -CₜH₂ₜOCOR⁵, n, m, p and t are the same or different and each represents an integer of 2 to 4, and q, r and s each represents an integer of 1 to 5.

Preferable examples of the compound (3') are represented by the following formulae:

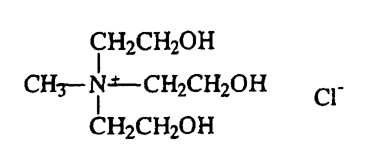

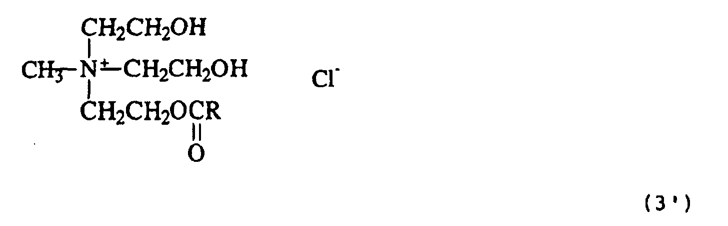

In the above shown formulae, RCO groups are, for example, those residues of hardened tallow fatty acid, tallow fatty acid, palm fatty acid, palm kernel oil fatty acid, palm stearic acid, oleic acid or industrial stearic acid from which a hydroxyl group was removed, or C₁₇H₃₅CO, C₁₁H₂₃CO, or mixtures thereof.

In the fatty acid anhydride (2) used in this invention, two R⁶ groups are the same or different and each represents a C₇₋₃₅ linear or branched alkyl or alkenyl group, preferably a C₁₁₋₂₂ alkyl or alkenyl group, particularly preferably that residue of tallow fatty acid, palm fatty acid, palm kernel oil fatty acid, palm stearic acid or a hardened fatty acid thereof from which a carboxyl group was removed.

The fatty acid anhydride (2) can be obtained, for example, by thermally dehydrating the corresponding fatty acid or by adding a dehydrating agent such as acetic anhydride to the fatty acid.

The quaternary ammonium salt having an ester group, produced in this invention, is a compound wherein one or more hydroxyl groups in the quaternary ammonium salt (1) are esterified, and esterification of all the hydroxyl groups is not always necessary. When a plurality of hydroxyl groups are present, its esterified product occurs as a mixture. That is, when 3 hydroxyl groups are present in the quaternary ammonium salt (1), the resulting esterified compound may be a mixture of triester, diester, monoester and non-ester.

Preferable examples of the quaternary ammonium salt having an ester group produced in this invention include: 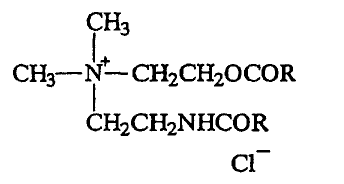 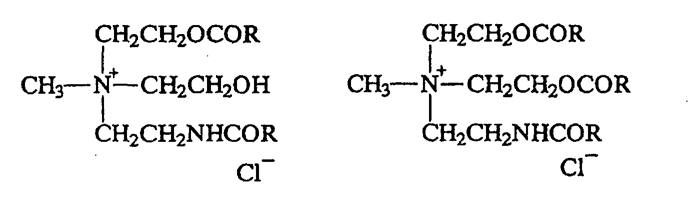 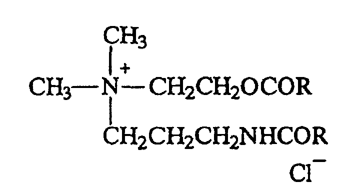 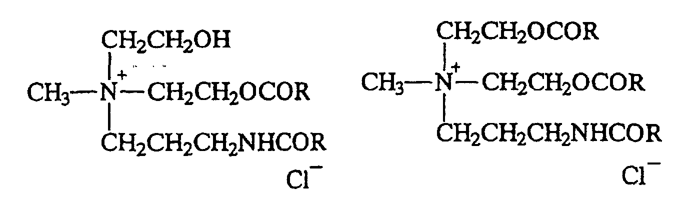 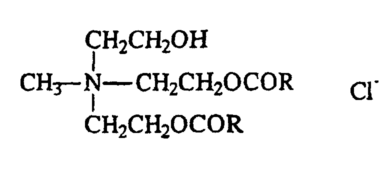 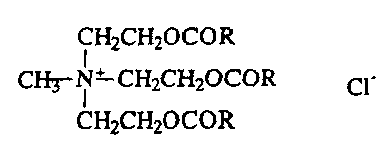 wherein RCO groups are, for example, those residues of hardened tallow fatty acid, tallow fatty acid, palm fatty acid, palm kernel oil fatty acid, palm stearic acid, oleic acid or industrial stearic acid from which a hydroxyl group was removed, or C₁₇H₃₅CO, C₁₁H₂₃CO, or mixtures thereof.

The esterification of the quaternary ammonium salt (1) with the fatty acid anhydride (2) is carried out preferably at a temperature ranging from the temperature where the quaternary ammonium salt(1) is in the molten state, the melting point of (1), to 150°C from the viewpoint of prevention of decomposition of the formed ester and the quaternary ammonium salt (1) as the starting material.

A solvent can be used to reduce such a melting point of the quaternary ammonium salt (1), but it may be not always used. When the solvent is used, it is preferably inert to the fatty acid anhydride (2). A solvent having neither hydroxyl group, nor primary amino group nor secondary amino group is especially preferable. When a solvent is used, the solvent can be distilled away under reduced pressure during or after the reaction, or after the quaternary ammonium salt (1) is reacted with the fatty acid anhydride (2), water or a lower alcohol such as methanol, ethanol or propanol is added to convert the unreacted fatty acid anhydride (2) completely into the corresponding fatty acid or its lower alcohol ester, and thereafter, the solvent may be distilled away with water or the lower alcohol. In this reaction, the fatty acid formed by esterification of the quaternary ammonium salt (1) with the fatty acid anhydride (2) may act as a solvent to improve handling properties of the quaternary ammonium salt having a formed ester group.

The molar ratio of the fatty acid anhydride (2) to the quaternary ammonium salt (1), that is, [(2)/(1)], is preferably 0.5 to 2.5, more preferably 0.5 to 1.5. By conducting the reaction in such a molar ratio, the degree of esterification can be 0.5 to 2.5, preferably 0.5 to 1.5. However, when the number of hydroxyl groups in the quaternary ammonium salt (1) is n, the molar ratio of the fatty acid anhydride (2) to the quaternary ammonium salt (1), that is, [(2)/(1)], can be n or less to minimize the fatty acid formed as a byproduct.

The reaction may be conducted by introducing the quaternary ammonium salt (1) and the fatty acid anhydride (2) simultaneously or by introducing one of the two compounds and then adding the other compound dropwise thereto. When a part of hydroxyl groups in the quaternary ammonium salt (1) is to be esterified, it is preferable that the quaternary ammonium salt (1) is first introduced and then the fatty acid anhydride (2) is added thereto. In the case a high melting point of the quaternary ammonium salt (1) makes the reaction difficult, it is preferred to add a solvent in order to convert the quaternary ammonium salt (1) in the form of liquid and then to add the fatty acid anhydride (2) thereto, which is advantageous because of easily controlling the reaction temperature. The solvent added may be distilled away if necessary as described above, but the step of distilling it away is not always necessary.

According to the process of this invention, a quaternary ammonium salt having a hydroxyl group can be esterified in a high degree of conversion to produce a quaternary ammonium salt having an ester group with less byproduct such as fatty acid.

### Examples

In the Examples and Comparative Examples below, the reaction products were analyzed by ¹H-NMR (CDCl₃ solvent) to determine the molar ratio of the quaternary ammonium salt (1) as the starting material to its esterified product. Other byproducts were also analyzed.

### Synthesis Example 1

A four-necked flask equipped with a stirrer, a thermometer, a condenser and a dehydration tube was charged with hardened tallow fatty acid (500 g, 0.1822 mol) and heated up to 100°C under stirring. Then, acetic anhydride (158.1 g, 1.549 mol) was added thereto, and the mixture was reacted under heating for 2.5 hours up to 200°C with passage of nitrogen, and then aged for 2 hours. The distilled fluid (acetic acid and acetic anhydride) was continuously removed. Thereafter, the hardened tallow fatty acid anhydride was collected by distillation at 100°C at a reduced pressure of 0.4 kPa. As a result of analyzing the reaction product by ¹H-NMR, the purity of the hardened tallow fatty acid anhydride was 95%.

### Example 1

A four-necked flask equipped with a stirrer, a thermometer, a condenser and a dehydration tube was charged with (2-hydroxyethyl)dimethyl(2-hardened tallow alkanoyl amide ethyl) ammonium chloride (hereinafter referred to as quaternary ammonium salt (4)) (71.2 g, 0.161 mol) represented by formula (4) below, toluene (54.8 g) and hardened tallow fatty acid anhydride (87.8 g, 0.164 mol), and the mixture was heated up to 123°C under stirring. After reaching 123°C. the reaction solution was aged for 6 hours and then cooled down to 100°C, and the toluene was distilled away for 3.5 hours at a reduced pressure of 0.4 kPa, to give dimethyl(2-hardened tallow alkanoyl amide ethyl)(2-hardened tallow alkanoyl oxyethyl) ammonium chloride (hereinafter referred to as quaternary ammonium salt (5)) represented by formula (5) below. As a result of analyzing the reaction product by ¹H-NMR, the molar ratio of the quaternary ammonium salt (4)/quaternary ammonium salt (5) was 0/100. 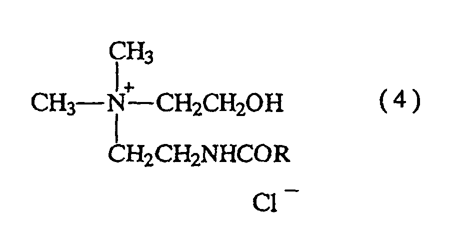 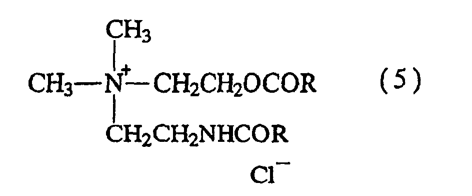 wherein RCO is a residue of hardened tallow fatty acid from which a hydroxyl group was removed.

### Example 2

A four-necked flask equipped with a stirrer, a thermometer, a condenser and a dehydration tube was charged with the quaternary ammonium salt (4) (71.2 g, 0.161 mol), toluene (54.8 g) and hardened tallow fatty acid anhydride (87.8 g, 0.164 mol), and the mixture was heated up to 123°C under stirring. After reaching 123°C, the reaction solution was aged for 6 hours to give the quaternary ammonium salt (5). As a result of analyzing the reaction product by ¹H-NMR, the molar ratio of the quaternary ammonium salt (4)/quaternary ammonium salt (5) was 0/100.

### Comparative Example 1

A four-necked flask equipped with a stirrer, a thermometer, a condenser and a dehydration tube was charged with the quaternary ammonium salt (4) (120 g, 0.118 mol) and hardened tallow fatty acid (64.6 g, 0.235 mol), and the mixture was heated up to 130°C under stirring. After reaching 130°C, the pressure in the flask was reduced to 0.5 kPa, and the reaction solution was aged for 6 hours to give the quaternary ammonium salt (5). As a result of analyzing the reaction product by ¹H-NMR, the molar ratio of the quaternary ammonium salt (4)/quaternary ammonium salt (5) was 85/15.

### Comparative Example 2

A four-necked flask equipped with a stirrer, a thermometer, a condenser and a dehydration tube was charged with the quaternary ammonium salt (4) (80.3 g, 0.182 mol), toluene (103.2 g) and hardened tallow fatty acid (100.0 g, 0.346 mol), and the mixture was heated to 130°C under stirring. Then, phosphoric acid (1.8 g) was added thereto, and the pressure in the flask was reduced down to 0.4 kPa. Further, the reaction solution was heated up to 150°C and aged for 6 hours . As a result of analyzing the reaction product by ¹H-NMR, this sample was found to consist of a mixture of the quaternary ammonium salt (5), the quaternary ammonium salt (4) (the quaternary ammonium salt (4) /quaternary ammonium salt (5) = 45/55 in terms of molar ratio), and a large mount of compounds of unknown structures (tertiary amine compounds).

### Synthesis Example 2

A 1-L autoclave was charged with 116.0 g dimethyl acetamide and 240.0 g (0.584 mol) of a dehydration reaction product of equimolar amounts of triethanolamine and hardened tallow fatty acid, and then 35.4 g (0.701 mol) methyl chloride was pressed into the autoclave. The mixture was reacted at 90°C for 6 hours, then cooled and removed. The hydroxyl value of the reaction product was 171.3. It was found in analyzing with ¹H-NMR that tri(2-hydroxyethyl)methylammonium chloride (6)/di(2-hydroxyethyl)methyl(2-hardened tallow alkanoyl oxyethyl)ammonium chloride (7)/(2-hydroxyethyl)methyldi(2-hardened tallow alkanoyl oxyethyl)ammonium chloride (8)/methyltri(2-hardened tallow alkanoyl oxyethyl)ammonium chloride (9) were present in the molar ratio of 36.6/50.2/13.2/0.0.

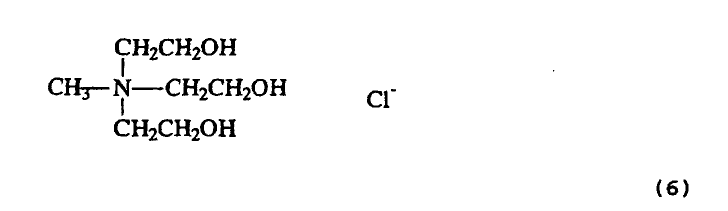

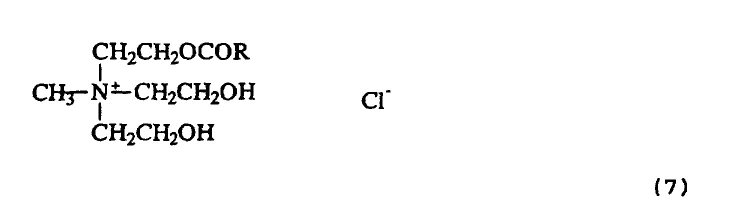

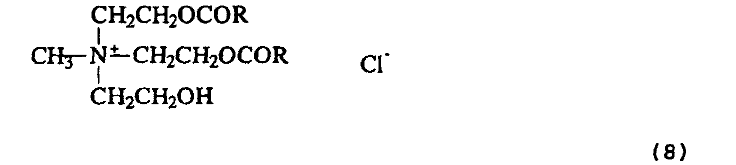

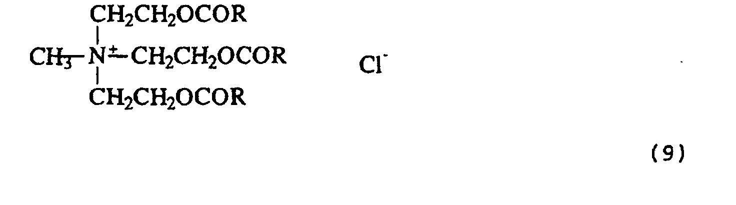

### Example 3

A four-necked flask equipped with a stirrer, a thermometer and a condenser was charged with the reaction product (40.7 g, 0.124 mol) in Synthesis Example 2 and hardened tallow fatty acid anhydride (purity 96.4%; 68.3 g, 0.124 mol), and the mixture was heated up to 90°C under stirring. After reaching 90°C, the reaction solution was aged for 6 hours. As a result of analyzing the reaction product by ¹H-NMR, (6)/(7)/(8)/(9) were present in the molar ratio of 0.0/4.8/17.9/77.3.

### Comparative Example 3

A four-necked flask equipped with a stirrer, a thermometer and a dehydrating tube was charged with the reaction product (40.7 g, 0.124 mol) in Synthesis Example 2 and hardened tallow fatty acid (166.5 g, 0.610 mol), and the mixture was heated to 90°C under stirring. After reaching 90°C. the pressure in the flask is reduced to 0.8 kPa, the reaction solution was aged for 6 hours. As a result of analyzing the reaction product by ¹H-NMR, formation of methyltri(2-hardened tallow alkanoyl oxyethyl)ammonium chloride(9) was not found.

## Claims

1. A process for producing a quaternary ammonium salt in which all or part of hydroxyl groups of the below shown quaternary ammonium salt (1) are esterified, which comprises reacting a quaternary ammonium salt (quaternary ammonium salt (1)) represented by formula (1) : 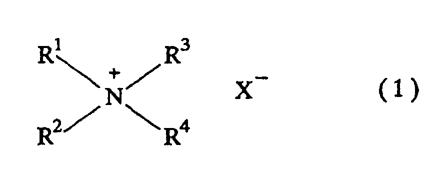 wherein X⁻ represents an anion, R¹ represents a C₁₋₆ alkyl group or a benzyl group, R² represents a C₁₋₆ alkyl group or -(CₙH₂ₙO)_{q}-H, R³ represents -(CₘH₂ₘO)ᵣ-H, R⁴ represents -(CₚH₂ₚO)₈-H or -CₜH₂ₜ-Y-COR⁵, n, m, p and t are the same or different and each represents an integer of 2 to 4, q, r and s each represents an integer of 1 to 5, Y represents -O- or -NH-, and R⁵ represents a C₇₋₃₅ linear or branched alkyl or alkenyl group,
with a fatty acid anhydride (fatty acid anhydride (2)) represented by formula (2) : 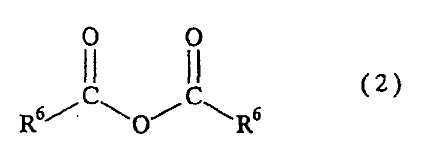 wherein R⁶ groups are the same or different and each represents a C₇₋₃₅ linear or branched alkyl or alkenyl group, in order to esterify all or a part of hydroxyl groups in the quaternary ammonium salt (1).

2. The process according to claim 1, wherein the reaction of the quaternary ammonium salt (1) with the fatty acid anhydride (2) is followed by adding water or a lower alcohol to convert the unreacted fatty anhydride (2) into the corresponding fatty acid or a lower alcohol ester thereof.

3. The process according to claim 1 or 2, wherein the quaternary ammonium salt (1) is a compound represented by formula (3) : 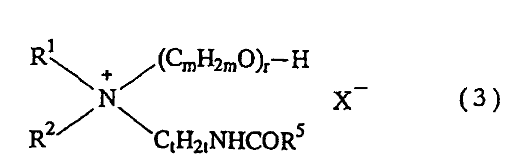 wherein X⁻, R¹, R², m, r, t and R⁵ have the same meanings as defined in claim 1.

4. The process according to claim 1 or 2, wherein in the formula (1) representing the quaternary ammonium salt (1), X⁻ represents an anion, R¹ represents a C₁₋₆ alkyl group or a benzyl group, R² represents -(CₙH₂ₙO)_{q}-H, R³ represents -(CₘH₂ₘO)ᵣ-H, R⁴ represents -(CₚH₂ₚO)ₛ-H or -CₜH₂ₜOCOR⁵, n, m, p and t are the same or different and each represents an integer of 2 to 4, and q, r and s each represents an integer of 1 to 5.

5. The process according to any one of claims 1 to 3, wherein in the fatty acid anhydride (2), R⁶CO groups are the same or different and each represents those residues of tallow fatty acid, palm fatty acid, palm kernel oil fatty acid, palm stearic acid or a hardened fatty acid thereof from which a hydroxyl group was removed.

## Patentansprüche

1. Verfahren zur Erzeugung eines quaternären Ammoniumsalzes, bei dem alle oder ein Teil der Hydroxylgruppen des unten gezeigten quaternären Ammoniumsalzes (1) verestert sind, umfassend die Umsetzung eines quaternären Ammoniumsalzes (quaternäres Ammoniumsalz (1)) der Formel (1): 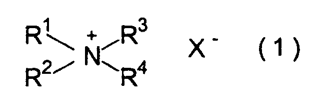 worin X⁻ ein Anion ist, R¹ eine C₁₋₆-Alkylgruppe oder eine Benzylgruppe ist, R² eine C₁₋₆-Alkylgruppe oder - (CₙH₂ₙO)_{q}-H ist, R³ -(CₘH₂ₘO)ᵣ-H ist, R⁴ -(CₚH₂ₚO)ₛ-H oder CₜH₂ₜ-Y-COR⁵ ist, worin n, m, p und t gleich oder verschieden voneinander sind und jeweils eine ganze Zahl von 2 bis 4 sind, q, r und s jeweils eine ganze Zahl von 1 bis 5 sind, Y -O- oder -NH- ist und R⁵ eine lineare oder verzweigte C₇₋₃₅-Alkyl- oder -Alkenylgruppe ist,
mit einem Fettsäureanhydrid (Fettsäureanhydrid (2)) der Formel (2): 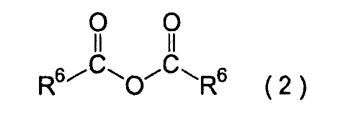 worin die Gruppen R⁶ gleich oder verschieden sind und jeweils eine lineare oder verzweigte C₇₋₃₅-Alkyloder -Alkenylgruppe sind, zur Veresterung eines Teils oder aller Hydroxylgruppen in dem quaternären Ammoniumsalz (1).

2. Verfahren nach Anspruch 1, worin die Umsetzung des quaternären Ammoniumsalzes (1) mit dem Fettsäureanhydrid von der Zugabe von Wasser oder einem niedrigen Alkohol gefolgt wird, zum Umwandeln des nicht-reagierten Fettsäureanhydrids (2) zu der entsprechenden Fettsäure oder einem Niedrigalkoholester davon.

3. Verfahren nach Anspruch 1 oder 2, worin das quaternäre Ammoniumsalz (1) eine Verbindung der Formel (3) ist: 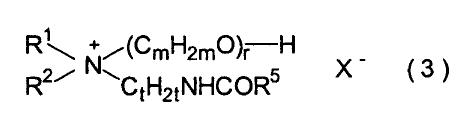 worin X⁻, R¹, R², m, r, t und R⁵ die gleichen Bedeutungen wie in Anspruch 1 definiert aufweisen.

4. Verfahren nach Anspruch 1 oder 2, worin in der Formel (1), die das quaternäre Ammoniumsalz (1) darstellt, X⁻ ein Anion, R¹ eine C₁₋₆-Alkylgruppe oder eine Benzylgruppe ist, R² -(CₙH₂ₙO)_{q}-H ist, R³ -(CₘH₂ₘO)ᵣ-H ist, R⁴ -(CₚH₂ₚO)ₛ-H oder CₜH₂ₜOCOR⁵ ist, n, m, p und t gleich oder verschieden voneinander sind und jeweils eine ganze Zahl von 2 bis 4 sind, und q, r und s jeweils eine ganze Zahl von 1 bis 5 sind.

5. Verfahren nach einem der Ansprüche 1 bis 3, worin in dem Fettsäureanhydrid (2) die Gruppen R⁶CO gleich oder verschieden voneinander sind und jeweils solche Reste von Talg-Fettsäure, Palm-Fettsäure, Palmkernöl-Fettsäure, Palmstearinsäure oder gehärteter Fettsäure davon bedeuten, von der eine Hydroxylgruppe entfernt ist.

## Revendications

1. Procédé pour la production d'un sel d'ammonium quaternaire dans lequel tous ou une partie des groupes hydroxyle du sel d'ammonium quaternaire (1) montré plus bas sont estérifiés, qui comprend de faire réagir un sel d'ammonium quaternaire (sel d'ammonium quaternaire (1)) représenté par la formule (1) : 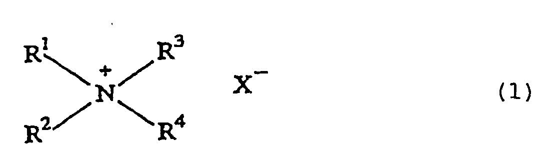 dans laquelle X⁻ représente un anion, R¹ représente un groupe alkyle en C₁₋₆ ou un groupe benzyle, R² représente un groupe alkyle en C₁₋₆ ou -(CₙH₂ₙO)_{q}-H, R³ représente -(CₘH₂ₘO)ᵣ-H, R⁴ représente -(CₚH₂ₚO)ₛ-H ou -CₜH₂ₜ-Y-COR⁵, n, m, p et t sont les mêmes ou différents et chacun représente un nombre entier de 2 à 4, q, r et s chacun représente un nombre entier de 1 à 5, Y représente -O- ou -NH-, et R⁵ représente un groupe alkyle ou alcényle linéaire ou ramifié en C₇₋₃₅,
avec un anhydride d'acide gras (anhydride d'acide gras (2)) représenté par la formule (2) : 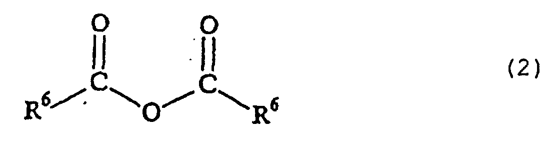 dans laquelle les groupes R⁶ sont les mêmes ou différents et chacun représente un groupe alkyle ou alcényle linéaire ou ramifié en C₇₋₃₅, de manière à estérifier tous ou une partie des groupes hydroxyle dans le sel d'ammonium quaternaire (1).

2. Procédé selon la revendication 1, dans lequel la réaction du sel d'ammonium quaternaire (1) avec l'anhydride d'acide gras (2) est suivie d'une addition d'eau ou d'un alcool inférieur afin de convertir l'anhydride gras non réagi (2) en l'acide gras correspondant ou un ester d'alcool inférieur de celui-ci.

3. Procédé selon la revendication 1 ou 2, dans lequel le sel d'ammonium quaternaire (1) est un composé représenté par la formule (3) : 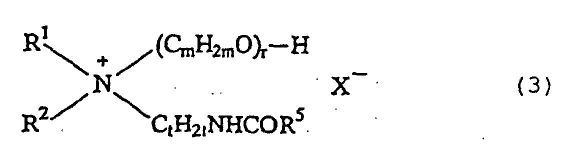 dans laquelle X⁻, R¹, R², m, r, t et R⁵ ont les mêmes significations que définies dans la revendication 1.

4. Procédé selon la revendication 1 ou 2, dans lequel dans la formule (1) représentant le sel d'ammonium quaternaire (1), X⁻ représente un anion, R¹ représente un groupe alkyle en C₁₋₆ ou un groupe benzyle, R² représente -(CₙH₂ₙO)_{q}-H, R³ représente -(CₘH₂ₘO)ᵣ-H, R⁴ représente -(CₚH₂ₚO)ₛ-H ou - CₜH₂ₜOCOR⁵, n, m, p et t sont les mêmes ou différents et chacun représente un nombre entier de 2 à 4, et q, r et s chacun représente un nombre entier de 1 à 5.

5. Procédé selon l'une quelconque des revendications 1 à 3, dans lequel dans l'anhydride d'acide gras (2), les groupes R⁶CO sont les mêmes ou différents et chacun représente des résidus d'acide gras de suif, d'acide gras de palme, d'acide gras d'huile de palmiste, d'acide stéarique de palme ou un acide gras durci de ceux-ci à partir desquels un groupe hydroxyle a été enlevé.
